(19) Europäisches Patentamt
European Patent Office
Office européen des brevets

(11) Veröffentlichungsnummer: 0 181 588
A2

(12) **EUROPÄISCHE PATENTANMELDUNG**

(21) Anmeldenummer: 85114019.4

(22) Anmeldetag: 05.11.85

(51) Int. Cl.⁴: C 07 D 215/56
A 61 K 31/47

(30) Priorität: 15.11.84 DE 3441788

(43) Veröffentlichungstag der Anmeldung: 21.05.86 Patentblatt 86/21

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Grohe, Klaus, Dr.
Am Wasserturm 10
D-5068 Odenthal(DE)

(72) Erfinder: Schriewer, Michael, Dr.
Heymannstrasse 36
D-5090 Leverkusen(DE)

(72) Erfinder: Zeiler, Hans-Joachim, Dr.
Elsbeekerstrasse 46
D-5620 Velbert 15(DE)

(72) Erfinder: Metzger, Karl Georg, Dr.
Pahlkestrasse 75
D-5600 Wuppertal(DE)

(54) **Alkyl-l-cyclopropyl-l,4-dihydro-4-oxo-3-chinolincarbonsäuren, Verfahren zu ihrer Herstellung sowie diese enthaltende antibakterielle Mittel.**

(57) Die Erfindung betrifft neue alkyl-1-cyclopropyl-1,4-dihydro-4-oxo-3-chinolincarbonsäuren, Verfahren zu ihrer Herstellung sowie diese enthaltende antibakterielle Mittel und ihre Verwendung bei der Bekämpfung von Krankheiten.

Croydon Printing Company Ltd.

BAYER AKTIENGESELLSCHAFT 5090 Leverkusen, Bayerwerk

Konzernverwaltung RP Ad/by-c
Patentabteilung

# Alkyl-1-cyclopropyl-1,4-dihydro-4-oxo-3-chinolincarbonsäuren, Verfahren zu ihrer Herstellung sowie diese enthaltende antibakterielle Mittel

Die Erfindung betrifft neue Alkyl-1-cyclopropyl-1,4-dihydro-4-oxo-3-chinolincarbonsäuren, Verfahren zu ihrer Herstellung sowie diese enthaltende antibakterielle Mittel und ihre Verwendung bei der Bekämpfung von Krankheiten.

7-Amino-1-cyclopropyl-4-oxo-1,4-dihydro-naphthyridin-3-carbonsäuren und 1-Cyclopropyl-6-fluor-1,4-dihydro-4-oxo-7-piperazino-chinolin-3-carbonsäuren sowie deren Verwendung als antibakterielle Mittel sind aus den DE-OSen 3 033 157 und 3 142 854 bekannt.

Gefunden wurden nun die neuen Alkyl-1-cyclopropyl-1,4-dihydro-4-oxo-3-chinolincarbonsäuren der Formel I

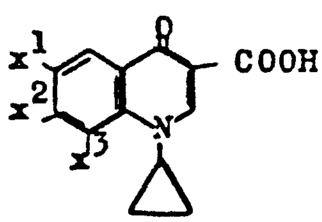

(I)

in welcher

$X^1$, $X^2$ und $X^3$ Wasserstoff und/oder einen Alkylrest mit 1 bis 3 Kohlenstoffatomen bedeuten mit der Maßgabe, daß zumindest ein Rest $X^1$, $X^2$ oder $X^3$ einen Alkylrest darstellt, und ferner $X^1$, $X^2$ und $X^3$ die Nitrogruppe oder ein Halogenatom, bevorzugt Fluor oder Chlor sein können

und deren pharmazeutisch verwendbare Salze.

Die Verbindungen der Formel I eignen sich als Wirkstoffe in der Human- und Veterinärmedizin, wobei zur Veterinärmedizin auch die vorbeugende Behandlung von Fischen zu zählen ist.

Die Alkyl-1-cyclopropyl-1,4-dihydro-4-oxo-3-chinolincarbonsäuren I können gemäß folgendem Reaktionsschema hergestellt werden:

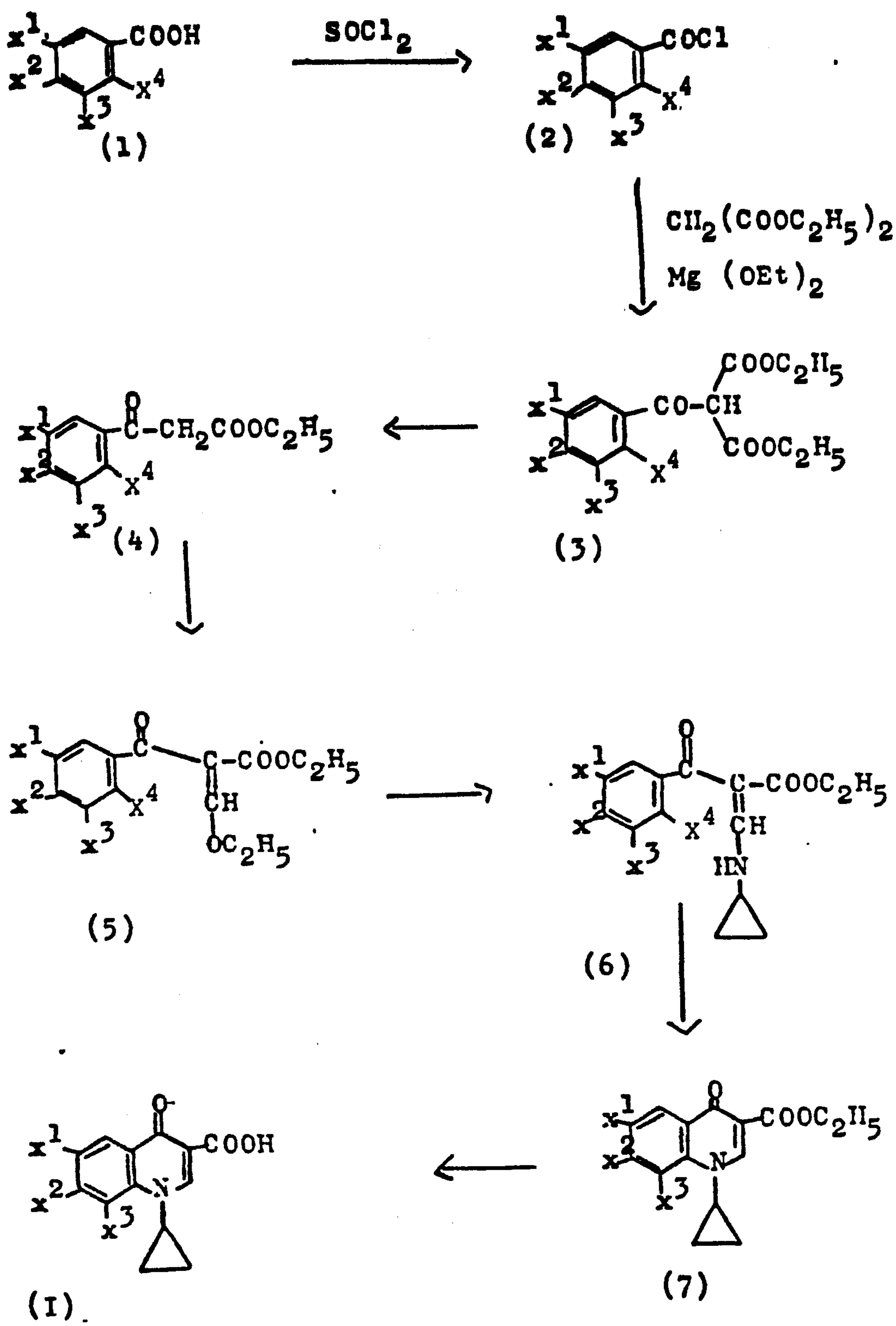


(1) (2) (3) (4) (5) (6) (7) (I)

wobei $X^4$ Halogen, bevorzugt Chlor oder Fluor und Nitro bedeutet.

Danach wird Malonsäurediethylester in Gegenwart von Magnesiumethylat mit dem substituierten Benzoylhalogenid (2) zum Aroylmalonester (3) acyliert (Organicum, 3. Auf. 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Aroylessigsäureethylester (4), der mit o-Ameisensäuretriethylester/Acetanhydrid in den substituierten 3-Ethoxyacrylsäureethylester (5) übergeht. Die Umsetzung von (5) mit Cyclopropylamin in einem Lösungsmittel, wie z.B. Methylenchlorid, Ethylalkohol, Chloroform, Cyclohexan oder Toluol führt in leicht exothermer Reaktion zum gewünschten Zwischenprodukt (6).

Die Cyclisierungsreaktion (6) ——— (7) wird in einem Temperaturbereich von etwa 60 bis 300°C, bevorzugt 80 bis 180°C durchgeführt.

Als Verdünnungsmittel für die Cyclisierungsreaktion können Dimethylsulfoxid, N-Methylpyrrolidon, Sulfolan, Hexamethylphosphorsäuretriamid und bevorzugt N,N-Dimethylformamid verwendet werden.

Als Säurebinder kommen für diese Reaktionsstufe Kalium-tert.-butanolat, Butyl-lithium, Lithium-phenyl, Phenylmagnesiumbromid, Natriummethylat, Natriumhydrid und

besonders bevorzugt Kalium- oder Natriumcarbonat in Betracht. Es kann dabei vorteilhaft sein, einen Überschuß von 10 Mol-% an Base einzusetzen.

Die im letzten Schritt erfolgende Esterhydrolyse von (7) unter basischen oder sauren Bedingungen führt zu den Alkyl-1-cyclopropyl-1,4-dihydro-4-oxo-3-chinolincarbonsäuren (I).

Die Erfindung betrifft demnach auch ein Verfahren zur Herstellung von Alkyl-1-cyclopropyl-1,4-dihydro-4-oxo-3-chinolincarbonsäuren der Formel I

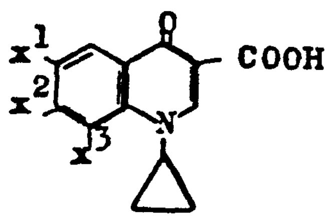

(I)

das dadurch gekennzeichnet ist, daß man substituierte Benzoesäuren der Formel (1)

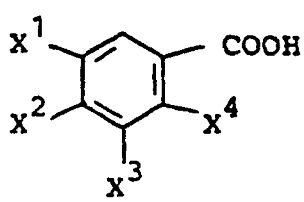

(1)

in der

$X^4$ für Nitro, Halogen, bevorzugt für Chlor oder Fluor steht und

$X^1$,$X^2$ und $X^3$ die oben angegebene Bedeutung haben,

in das entsprechende Säurehalogenid der Formel (2)

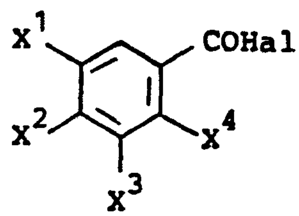

(2)

worin

$X^1$,$X^2$,$X^3$ und $X^4$ wie oben definiert sind und

Hal für Halogen, bevorzugt für Cl steht,

beispielsweise durch Umsetzung mit $SOCl_2$ überführt.

Malonsäurediethylester in Gegenwart von Magnesiummethylat mit dem substituierten Benzoylhalogenid (2) zum Aroylmalonester der Formel (3)

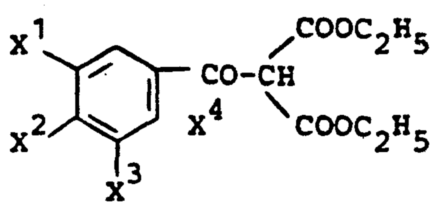

(3)

acyliert, wobei

$X^1$,$X^2$,$X^3$ und $X^4$ wieder wie oben definiert sind,

den Aroylmalonester (3) durch partielle Verseifung und Decarboxylierung in wäßrigem Medium mit katalytischen Mengen Schwefelsäure oder p-Toluolsulfonsäure in den Aroylessigsäureethylester (4)

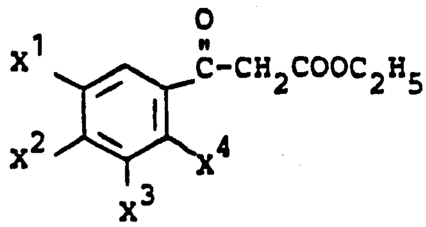

(4)

überführt, wobei

$X^1, X^2, X^3$ und $X^4$ wieder wie oben definiert sind,

den Aroylessigsäureethylester mit o-Ameisentriethylester/ Acetanhydrid zum substituierten 3-Ethoxy-acrylsäureethylester (5)

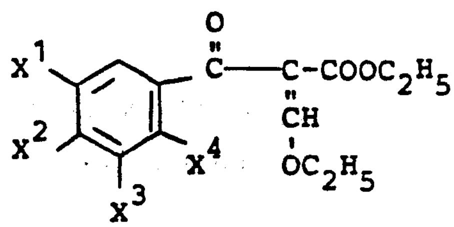

(5)

mit den Substituentendefinitionen wie oben umsetzt,

das Umsetzungsprodukt (5) mit Cyclopropylamin in einem Lösungsmittel wie Methylenchlorid, Alkohol, Chloroform, Cyclohexan oder Toluol in das Zwischenprodukt (6)

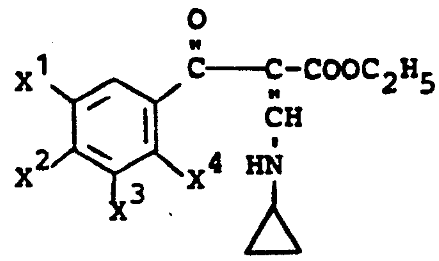

(6)

mit den Substituentendefinitionen wie oben überführt,

das Zwischenprodukt bei Temperaturen von 60 bis 300°C zur Verbindung (7)

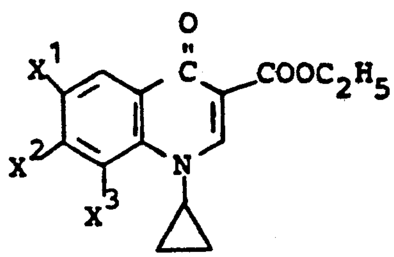

(7)

cyclisiert und schließlich die Verbindung (7) zu Verbindungen der Formel (I) verseift.

Verwendet man 2-Chlor-3,5-difluor-4-methylbenzoylchlorid als Ausgangsstoff, so kann der Reaktionsablauf durch das folgende Formelschema wiedergegeben werden:

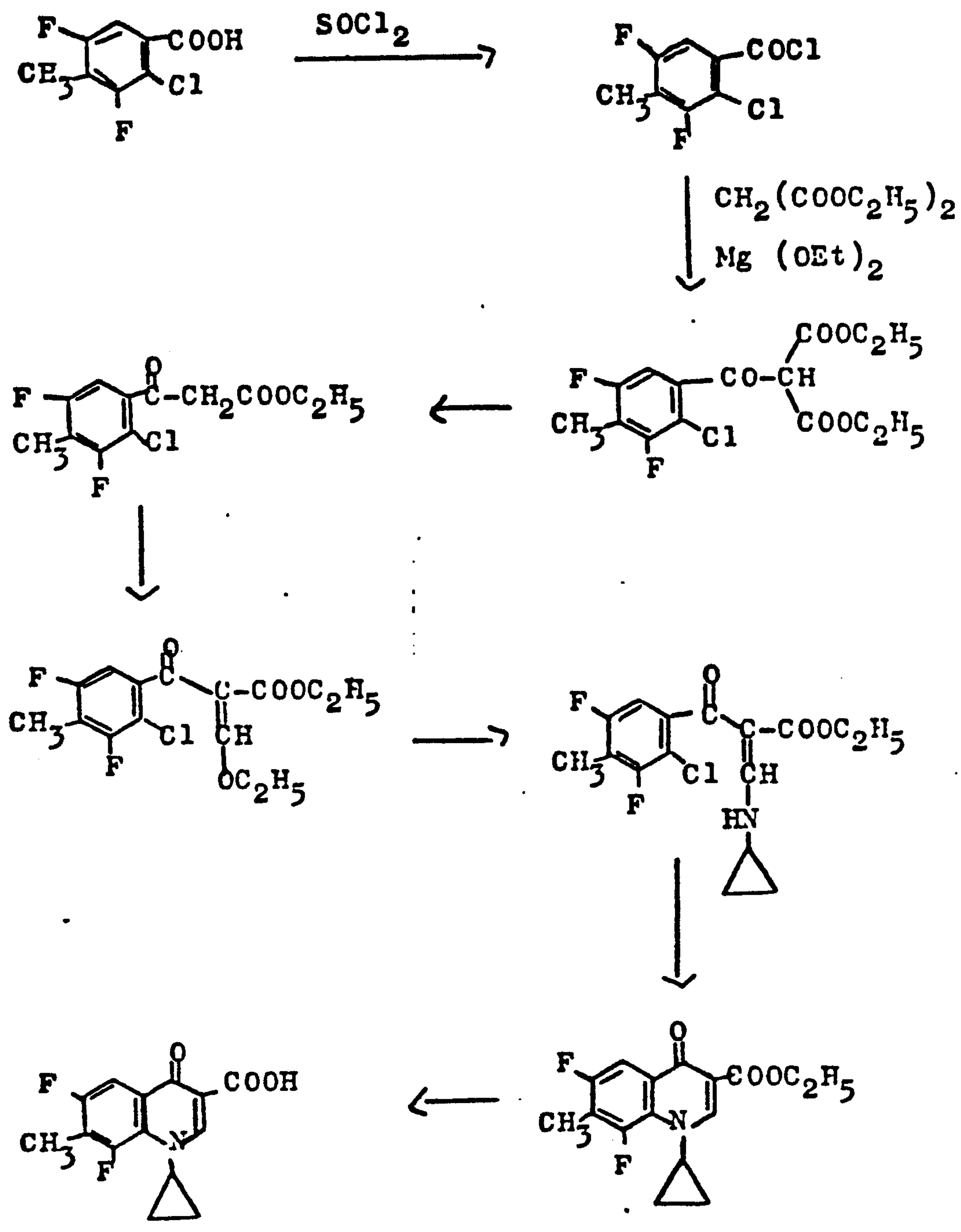
SOCl2
CH2(COOC2H5)2
Mg (OEt)2

Folgende als Ausgangsmaterialien verwendeten Benzoylhalogenide oder die korrespondierenden Benzoesäuren sind in der Literatur beschrieben:

2,5-Dichlor-4-methylbenzoesäurechlorid,
5-Brom-2-chlor-4-methylbenzoesäurechlorid,
2-Chlor-3,5-dinitro-4-methylbenzoesäurechlorid,
2,4-Dinitro-5-methylbenzoesäurechlorid,
2,5-Dichlor-4-methyl-3-nitrobenzoesäurechlorid,
2,4-Dichlor-3-methylbenzoesäurechlorid,
2,4-Dichlor-5-methylbenzoesäurechlorid,
2-Chlor-4,5-dimethylbenzoesäurechlorid,
2-Chlor-4-methyl-5-nitrobenzoesäurechlorid.

Die Synthesen der neuen Benzoylhalogenide sind im expermentellen Teil beschrieben.

Als neue Wirkstoffe seien im einzelnen genannt:

1-Cyclopropyl-6,8-difluor-7-methyl-1,4-dihydro-4-oxo-3-chinolincarbonsäure, 1-Cyclopropyl-6-fluor-7-methyl-1,4-dihydro-4-oxo-3-chinolincarbonsäure, 6-Chlor-1-cyclopropyl-7-methyl-1,4-dihydro-4-oxo-3-chinolincarbonsäure, 7-Chlor-1-cyclopropyl-8-methyl-1,4-dihydro-4-oxo-3-chinolincarbonsäure, 8-Chlor-1-cyclopropyl-6-fluor-7-methyl-1,4-dihydro-4-oxo-3-chinolincarbonsäure, 6-Chlor-1-cyclopropyl-8-fluor-7-methyl-1,4-dihydro-4-oxo-3-chinolincarbonsäure, 7-Chlor-1-cyclopropyl-6-fluor-8-methyl-1,4-dihydro-4-oxo-3-chinolincarbonsäure, 1-Cyclopropyl-6-fluor-7-methyl-8-nitro-1,4-dihydro-4-oxo-3-chinolin-

carbonsäure, 1-Cyclopropyl-7-methyl-6,8-dinitro-1,4-dihydro-4-oxo-3-chinolincarbonsäure, 1-Cyclopropyl-6-methyl-7-nitro-1,4-dihydro-4-oxo-3-chinolincarbonsäure, 6-Chlor-1-cyclopropyl-7-methyl-8-nitro-1,4-dihydro-4-oxo-3-chinolincarbonsäure, 7-Chlor-1-cyclopropyl-6-methyl-1,4-dihydro-4-oxo-3-chinolincarbonsäure, 1-Cyclopropyl-6,7-dimethyl-1,4-dihydro-4-oxo-3-chinolincarbonsäure, 1-Cyclopropyl-6,7-dimethyl-8-nitro-1,4-dihydro-4-oxo-3-chinolincarbonsäure, 1-Cyclopropyl-7-methyl-6-nitro-1,4-dihydro-4-oxo-3-chinolincarbonsäure und deren pharmazeutisch verwendbaren Alkalisalze, Erdalkalisalze oder Hydrate.

Die erfindungsgemäßen Verbindungen besitzen hervorragende antimikrobielle Eigenschaften.

Insbesondere sind sie breit bakteriostatisch und bakterizid wirksam gegen grampositive Bakterien, wie Staphylokokken und Streptokokken, und gramnegative Bakterien wie Escherichia, Proteus, Providencia, Enterobacter, Klebsiella, Salmonella und Pseudomonas. Die Aufzählung empfindlicher Bakterien ist beispielhaft und keineswegs einschränkend zu sehen.

Die verbesserte breite antibakterielle Wirksamkeit der erfindungsgemäßen Verbindungen ermöglicht ihren Einsatz als Wirkstoffe sowohl in der Human- als auch in der Veterinärmedizin, wobei sie sowohl zur Verhütung als auch zur Behandlung von systemischen oder lokalen bakteriellen Infektionen, insbesondere der Harnwege

verwendet werden können. Die erfindungsgemäßen Verbindungen können weiterhin auch als Futterzusatzmittel zur Förderung des Wachstums und zur Verbesserung der Futterauswertung in der Tierhaltung, insbesondere bei der Haltung von Mastvieh, verwendet werden. Die Applikation der Wirkstoffe erfolgt dann vorzugsweise über das Futter und/oder das Trinkwasser.

Die vorliegende Erfindung betrifft weiterhin Mittel, die die neuen erfindungsgemäßen Verbindungen enthalten sowie die Herstellung dieser Mittel. Hierzu gehören beispielsweise Futtermittelkonzentrate für die Tierhaltung, die in üblicher Weise neben den Wirkstoffen auch Vitamine und/oder Mineralsalze enthalten können oder pharmazeutische Zubereitungen.

Die Erfindung betrifft bevorzugt antibakteriell wirksame Mittel, die Verbindungen der Formel I enthalten. Die Erfindung betrifft besonders bevorzugt solche antibakteriell wirksamen Mittel, die Verbindungen der Formel I oder deren Alkali- oder Erdalkalisalze enthalten.

Die erfindungsgemäßen pharmazeutischen Zubereitungen enthalten neben den neuen erfindungsgemäßen Verbindungen in üblicher Weise nicht toxische, inerte pharmazeutisch geeignete Trägerstoffe. Solche pharmazeutisch geeignete Trägerstoffe sind beispielsweise Füll- und Streckmittel, Bindemittel, Feuchthaltemittel, Lösungsverzögerer, Resorptionsbeschleuniger, Netzmittel, Adsorbtionsmittel oder Gleitmittel, die feste, halbfeste oder flüssige Konsistenz haben können. Solche pharmazeutisch geeignete Trägerstoffe sind dem Fachmann bekannt.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotionen, Puder und Sprays genannt. Die Herstellung dieser Zubereitungen geschieht nach bekannten Methoden in üblicher Weise, beispielsweise durch Mischen des neuen erfindungsgemäßen Wirkstoffes mit den üblichen Träger- und Zusatzstoffen. Der Wirkstoff soll in den aufgeführten pharmazeutischen Zubereitungen in einer Konzentration von etwa 0,1 bis 99,5, vorzugsweise von etwa 0,5 bis 95 Gew.-% der Gesamtmischung vorhanden sein.

Die Bereitstellung neuer Bakterizide zur Bekämpfung von Bakterien, die gegen bekannte Bakterizide resistent sind, ist eine Bereicherung des Standes der Technik.

In der nachfolgenden Tabelle wird die Wirksamkeit einiger erfindungsgemäßer Wirkstoffe im MHK-Test nachgewiesen.

Tabelle 1

| Beispiel-Nr. | 1 | 2 | 12 | 6 |
|---|---|---|---|---|
| E. coli 4418 | 0,06 | ≤0,015 | ≤0,015 | ≤0,015 |
| E. coli Neumann | ≤0,015 | ≤0,015 | ≤0,015 | ≤0,015 |
| E. coli A261 | 0,06 | 0,015 | ≤0,015 | ≤0,015 |
| Klebsiella pneum. 63 | 0,25 | 0,125 | 0,03 | 0,03 |
| Klebsiella pneum. 8085 | 0,06 | ≤0,015 | 0,03 | ≤0,015 |
| Proteus vulgar. 1017 | 0,25 | ≤0,015 | 0,06 | ≤0,015 |
| Providencia stuartii 12012 | 0,25 | 0,03 | 0,06 | 0,03 |
| Styphylococcus aureus 133 | 0,25 | 2 | 0,125 | ≤0,015 |
| Staphylococcus aureus 1756 | 0,5 | 2 | 0,125 | ≤0,015 |
| Pseudomonas aeruginosa W. | 4 | 8 | 2 | 1 |

Agarverdünnungstest: Isosensitestmedium

Denley Multipoint-Inoculator

0181588

Herstellungsbeispiele

Beispiel 1

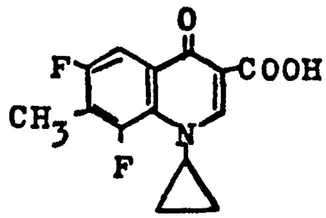

4,5 g Magnesiumspäne werden in 10 ml wasserfreiem Ethanol suspendiert. Man versetzt mit 1 ml Tetrachlorkohlenstoff und tropft, wenn die Reaktion in Gang gekommen ist, ein Gemisch von 29,7 g Malonsäurediethylester, 20 ml abs. Ethanol und 80 ml wasserfreiem Toluol bei 50-60°C zu. Dann wird noch 1 Stunde auf 50-60°C erhitzt, mit Trockeneis/Aceton auf -5°C bis -10°C gekühlt und bei dieser Temperatur eine Lösung von 41,7 g 2-Chlor-3,5-difluor-4-methylbenzoylchlorid in 20 ml abs. Toluol langsam zugetropft. Man rührt 1 Stunde bei 0 bis -5°C, läßt über Nacht auf Raumtemperatur kommen und läßt unter Eiskühlung ein Gemisch von 80 ml Eiswasser und 12 ml konz. Schwefelsäure zulaufen. Die Phasen werden getrennt und zweimal mit Toluol nachextrahiert. Die vereinigten Toluollösungen werden mit gesättigter NaCl-Lösung gewaschen, mit $Na_2SO_4$ getrocknet und das Lösungsmittel im Vakuum abgezogen. Man erhält 65,1 g 2-Chlor-3,5-difluor-4-methylbenzoyl-malonsäurediethylester als Rohprodukt.

Eine Emulsion von 65,1 g rohem 2-Chlor-3,5-difluor-4-methyl-benzoyl-malonsäurediethylester in 70 ml Wasser wird mit 0,1 g p-Toluolsulfonsäure versetzt. Man erhitzt unter gutem Rühren 4,5 Stunden zum Sieden, extrahiert die erkaltete Emulsion mehrmals mit Methylenchlorid, wäscht die vereinigten $CH_2Cl_2$-Lösungen einmal mit gesättigter NaCl-Lösung, trocknet mit $Na_2SO_4$ und destilliert das Lösungsmittel im Vakuum ab. Die Fraktionierung des Rückstandes im Feinvakuum liefert 33 g 2-Chlor-3,5-difluor-4-methylbenzoyl-essigsäureethylester vom Siedepunkt 108-118°C/0,09 mbar.

Ein Gemisch von 32,5 g 2-Chlor-3,5-difluor-4-methylbenzoylessigsäureethylester, 26,7 g o-Ameisensäureethylester und 30,6 g Acetanhydrid wird 2 Stunden auf 150°C erhitzt. Dann werden im Wasserstrahlvakuum und zuletzt im Feinvakuum bei einer Badtemperatur von ~120°C die flüchtigen Bestandteile abdestilliert. Zurück bleiben 38,6 g roher 2-(2-Chlor-3,5-difluor-4-methyl-benzoyl)-3-ethoxyacrylsäureethylester. Er ist genügend rein für die weiteren Umsetzungen.

Eine Lösung von 38,7 g 2-(2-Chlor-3,5-difluor-4-methylbenzoyl)-3-ethoxyacrylsäureethylester in 100 ml Ethanol wird unter Eiskühlung und Rühren tropfenweise mit 7,4 g Cyclopropylamin versetzt. Wenn die exotherme Reaktion abgeklungen ist, wird noch 1/2 Stunde bei Raumtemperatur gerührt, mit 110 ml Wasser versetzt, der Niederschlag kalt abgesaugt und im Vakuum getrocknet. Man erhält 38,2 g 2-(2-Chlor-3,5-difluor-4-methyl-benzoyl)-3-cyclopropyl-amino-acrylsäureethylester vom Schmelzpunkt 121-122°C.

Eine Lösung von 37,7 g 2-(2-Chlor-3,5-difluor-4-methyl-benzoyl)-3-cyclopropylamino-acrylsäureethylester in 100 ml wasserfreiem Dimethylformamid wird mit 16,5 g Kaliumcarbonat versetzt. Dann wird 2 Stunden unter Rückfluß gerührt und das Reaktionsgemisch heiß auf Eis gegossen. Der Niederschlag wird abgesaugt, mit Wasser gut gewaschen und im Vakuum über Calciumchlorid bei 100°C getrocknet. Man erhält 30,4 g 1-Cyclopropyl-6,8-difluor-7-methyl-1,4-dihydro-4-oxo-3-chinolincarbonsäure-ethylester vom Schmelzpunkt 177-179°C.

Ein Gemisch von 30 g 1-Cyclopropyl-6,8-difluor-7-methyl-1,4-dihydro-4-oxo-3-chinolincarbonsäureethylester, 210 ml Eisessig, 160 ml Wasser und 23,5 ml konz. Schwefelsäure wird 2 Stunden auf Rückfluß erhitzt. Dann gießt man die heiße Suspension auf Eis, saugt den Niederschlag ab, wäscht gut mit Wasser nach und trocknet im Vakuum bei 100°C. Man erhält auf diese Weise 25,9 g reine 1-Cyclopropyl-6,8-difluor-7-methyl-1,4-dihydro-4-oxo-3-chinolincarbonsäure vom Schmelzpunkt 227-229°C.

Beispiel 2

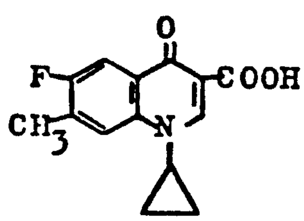

Analog Beispiel 1 erhält man ausgehend vom 2,5-Difluor-4-methyl-benzoylchlorid in vergleichbaren Ausbeuten den

2,5-Difluor-4-methyl-benzoyl-essigsäureethylester vom Siedepunkt 120-130°C/0,09 mbar, den 2-(2,5-Difluor-4-methyl-benzoyl)-3-cyclopropylamino-acrylsäureethylester vom Schmelzpunkt 65-67°C, den 1-Cyclopropyl-6-fluor-7-methyl-1,4-dihydro-4-oxo-3-chinolincarbonsäureethylester vom Schmelzpunkt 221-223°C und die 1-Cyclopropyl-6-fluor-7-methyl-1,4-dihydro-4-oxo-3-chinolin-3-carbonsäure vom Schmelzpunkt 246-248°C.

Analog Beispiel 1 wurden folgende 2-Benzoyl-3-cyclopropylamino-acrylsäureethylester erhalten:

Tabelle 2

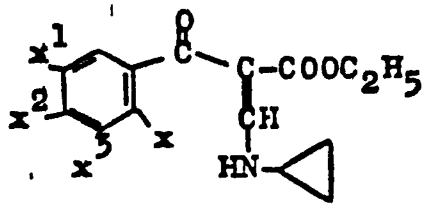

| $X^1$ | $X^2$ | $X^3$ | X | Schmelzpunkt (°C) |
|---|---|---|---|---|
| Cl | $CH_3$ | H | Cl | 108-110 |
| Br | $CH_3$ | H | Cl | 102-105 |
| Cl | $CH_3$ | F | Cl | 99-101 |
| F | $CH_3$ | Cl | Cl | 105-107 |
| F | Cl | $CH_3$ | Cl | 108-110 |
| F | $CH_3$ | $NO_2$ | Cl | 156-157 |
| $NO_2$ | $CH_3$ | $NO_2$ | Cl | 153-155 |
| $CH_3$ | $NO_2$ | H | $NO_2$ | 70-75 |
| Cl | $CH_3$ | $NO_2$ | Cl | 144-148 |
| H | Cl | $CH_3$ | Cl | 164-167 |
| $CH_3$ | Cl | H | Cl | 100-103 |
| $CH_3$ | $CH_3$ | H | Cl | 93-94 |
| $CH_3$ | $CH_3$ | $NO_2$ | Cl | 159-160 |
| $NO_2$ | $CH_3$ | H | Cl | 130-131 |

Die 2-Benzoyl-3-cyclopropylamino-acrylsäureester wurden analog Beispiel 1 in folgende 1-Cyclopropyl-1,4-dihydro-4-oxo-3-chinolincarbonsäureethylester übergeführt:

Tabelle 3

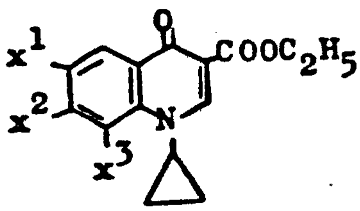

| $X^1$ | $X^2$ | $X^3$ | Schmelzpunkt (°C) |
|---|---|---|---|
| Cl | $CH_3$ | H | 218-220 |
| Br | $CH_3$ | H | 191-196 |
| Cl | $CH_3$ | F | 180-185 |
| F | $CH_3$ | Cl | 126-130 |
| F | Cl | $CH_3$ | 170-175 |
| F | $CH_3$ | $NO_2$ | 157-158 |
| $NO_2$ | $CH_3$ | $NO_2$ | 232-233 |
| $CH_3$ | $NO_2$ | H | 177-178 |
| Cl | $CH_3$ | $NO_2$ | 225-227 |
| H | Cl | $CH_3$ | 151-155 |
| $CH_3$ | Cl | H | 160-162 |
| $CH_3$ | $CH_3$ | H | 158-160 |
| $CH_3$ | $CH_3$ | $NO_2$ | 218-221 |
| $NO_2$ | $CH_3$ | H | 262-263 |

Die Verseifung analog Beispiel 1 führt zu den entsprechenden 1-Cyclopropyl-1,4-dihydro-4-oxo-3-chinolincarbonsäuren:

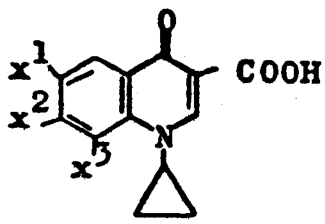

| Bsp. Nr. | $X^1$ | $X^2$ | $X^3$ | Schmelzpunkt (°C) |
|---|---|---|---|---|
| 3 | Cl | $CH_3$ | H | 260-262 |
| 4 | Br | $CH_3$ | H | 280-283 |
| 5 | Cl | $CH_3$ | F | 215-217 |
| 6 | F | $CH_3$ | Cl | 188-189 |
| 7 | F | Cl | $CH_3$ | 191-195 |
| 8 | F | $CH_3$ | $NO_2$ | 261-263 |
| 9 | $NO_2$ | $CH_3$ | $NO_2$ | 250-252 |
| 10 | $CH_3$ | $NO_2$ | H | 236-238 |
| 11 | Cl | $CH_3$ | $NO_2$ | 263-266 |
| 12 | H | Cl | $CH_3$ | 270-272 |
| 13 | $CH_3$ | Cl | H | 248-250 |
| 14 | $CH_3$ | $CH_3$ | H | 280-282 |
| 15 | $CH_3$ | $CH_3$ | $NO_2$ | 271-273 |
| 16 | $NO_2$ | $CH_3$ | H | 264-266 |

Die als Ausgangsstoffe verwendeten neuen Alkylbenzoesäuren wurden wie folgt erhalten:

Darstellung von 2,3-Dichlor-5-fluor-4-methyl-benzoesäure

1. 2-Chlor-5-fluor-4-methylbenzoesäure

200 ml 1,2-Dichlorethan und 80 g $AlCl_3$ werden unter Eiskühlung mit 41 g Acetylchlorid versetzt. Danach werden 72,3 g 5-Chlor-2-fluortoluol zugetropft. Man erwärmt 8 Stunden auf 50° und gibt dann das Reaktionsgemisch auf Eis. Die organische Phase wird mit Wasser gewaschen, getrocknet und destilliert. Ausbeute ca. 50 g 2-Chlor-5-fluor-4-methylacetophenon $Kp_{10}$ 120-127°.

In eine aus 80 g NaOH und 96 g Brom in 400 ml Wasser bereitete NaOBr-Lösung wird bei 0°C eine Lösung von 37,2 g 2-Chlor-5-fluor-4-methylacetophenon in 280 ml Dioxan getropft. Nach beendeter Reaktion wird das Bromoform abgetrennt. Die wäßrige Phase wird mit 20 g $Na_2S_2O_5$ versetzt und dann mit konz. HCl angesäuert. Dabei fallen 29,1 g 2-Chlor-5-fluor-4-methylbenzoesäure aus. Fp. 176-77°.

2. 2-Chlor-5-fluor-4-methyl-3-nitro-benzoesäure

20 g 2-Chlor-5-fluor-4-methylbenzoesäure werden in 60 ml konz. $H_2SO_4$ vorgelegt. Unter Eiskühlung werden 12,3 g $KNO_3$ portionsweise zugegeben. Danach wird 2 Stunden auf 50° erwärmt und dann auf Eis gegossen. Nach Isolierung und Umkristallisation aus Toluol werden 18,8 g 2-Chlor-5-fluor-4-methyl-3-nitrobenzoesäure von Fp. 186-88° erhalten.

3. 3-Amino-2-chlor-5-fluor-4-methyl-benzoesäure

18,5 g 2-Chlor-5-fluor-4-methyl-3-nitro-benzoesäure und 51,5 g $Na_2S_2O_4$ werden in einem Gemisch aus 160 ml Glykolmonomethylether und 160 ml Wasser 3 Stunden gekocht. In die noch warme Lösung werden 230 ml 1/2 konz. HCl gegeben. Man kocht noch einmal auf, kühlt ab und gießt in 560 ml Wasser. Der ausgefallene Feststoff wird isoliert und getrocknet. Ausbeute 12,4 g Fp. 182-84°.

4. 3-Amino-2-chlor-5-fluor-4-methyl-benzoesäuremethylester

12 g 3-Amino-2-chlor-5-fluor-4-methylbenzoesäure werden in 60 ml MeOH vorgelegt. Man leitet 20 Minuten HCl-Gas ein und kocht danach 5 Stunden am Rückfluß. Danach wird in Wasser gegossen, mit Soda alkalisch gestellt und das Produkt isoliert. Ausbeute 8 g Fp. 51-52°.

5. 2,3-Dichlor-5-fluor-4-methylbenzoesäure

8 g 3-Amino-2-chlor-5-fluor-4-methylbenzoesäuremethylester werden in wäßriger Lösung mit $NaNO_2$/HCl diazotiert. Diese Diazoniumsalzlösung wird zu einer Lösung von 4,1 g CuCl in 16 ml HCl konz. getropft. Danach wird erwärmt, bis kein Gas mehr entsteht. Nach Abkühlen wird der ausgefallene Feststoff abgetrennt und in 30 ml 50 %igem EtOH aufge-

nommen. Nach Zugabe von 2,3 g NaOH wird 1 Stunde gekocht. Nach Abkühlen auf Raumtemperatur säuert man mit HCl an und isoliert den ausgefallenen Feststoff. Fp.: 179-80°. Ausbeute 5,2 g.

Darstellung von 2-Chlor-4,5-dimethyl-3-nitro-benzoesäure

31,6 g 2-Chlor-4,5-dimethylbenzoesäure werden in 94 ml $H_2SO_4$ vorgelegt. Unter Eiskühlung werden 19 g $KNO_3$ portionsweise zugegeben. Man erwärmt anschließend noch 2 Stunden auf 50° und gießt dann auf Eis. Das Produkt wird isoliert und aus Toluol umkristallisiert. Ausbeute 25,3 g, Fp. 160-63°.

Darstellung von 2,4-Dichlor-5-fluor-3-methylbenzoesäure

1. 2,4-Dichlor-3-methyl-5-nitrobenzoesäure

30 g 2,4-Dichlor-3-methylbenzoesäure werden in 83 ml konz. $H_2SO_4$ vorgelegt. Unter Eiskühlung werden 16,7 g $KNO_3$ portionsweise zugegeben. Man erwärmt anschließend noch 2 Stunden auf 50° und gießt dann auf Eis. Die Nitroverbindung wird isoliert und aus Toluol umkristallisiert. Fp. 152-4°, Ausbeute 24 g.

2. 5-Amino-2,4-dichlor-3-methylbenzoesäure

55 g 2,4-Dichlor-3-methyl-5-nitrobenzoesäure und

141,6 g $Na_2S_2O_4$ werden in einem Gemisch aus 440 ml Glykolmonomethylether und 440 ml Wasser 3 Stunden gekocht. Zu der noch warmen Lösung gibt man 620 ml 1/2 konz. HCl und kocht dann noch einmal auf. Nach Abkühlen auf Raumtemperatur gießt man in 1,5 l Wasser und stellt mit Soda auf pH 5 ein. Es werden 24,6 g Aminobenzoesäure erhalten. Fp. 202-3°.

3. 5-Amino-2,4-dichlor-3-methylbenzoesäure-methylester

24 g 5-Amino-2,4-dichlor-3-methylbenzoesäure werden in 110 ml Methanol vorgelegt. Man leitet 20 Minuten HCl-Gas ein und kocht anschließend 5 Stunden am Rückfluß. Danach gießt man in Wasser und stellt mit Soda alkalisch. Es werden 24 g Ester vom Fp. 86-88° erhalten.

4. 2,4-Dichlor-5-fluor-3-methylbenzoesäure

24 g 5-Amino-2,4-dichlor-3-methylbenzoesäuremethylester werden in wäßriger Lösung mit $NaNO_2$/HCl diazotiert. Zu der Diazoniumsalzlösung gibt man bei 0°C 26 ml 30 %ige wäßrige $HBF_4$-Lösung. Das Reaktionsgemisch wird 30 Minuten bei 0° gehalten. Danach isoliert man das Tetrafluoroborat und trocknet es über $P_2O_5$. Ausbeute 23,5 g.

Das getrocknete Tetrafluoroborat wird im Kolben trocken zersetzt. Nach beendeter Reaktion fügt man

dem flüssigen Rückstand 100 ml 50 %igen EtOH und 7,9 g KOH zu und kocht 1 Stunde. Nach Abkühlen wird mit HCl sauer gestellt und der Feststoff isoliert. Nach Verrühren mit Ligroin werden 14,3 g Produkt vom Fp. 145-49° erhalten.

Synthese von 2,5-Dichlor-3-fluor-4-methylbenzoesäure

1. 2,5-Dichlor-4-methyl-3-nitrobenzoesäure

In ein Gemisch von 100 ml $H_2SO_4$ konz. und 20 ml 68 %iger $HNO_3$ werden portionsweise 41 g 2,5-Dichlor-4-methylbenzoesäure gegeben. Es wird 3 Stunden bei 50° gehalten und dann auf 600 g Eis gegeben. Der ausgefallene Feststoff wird isoliert, getrocknet und mit wenig Toluol verrührt. Fp. 210-18°, Ausbeute 43,8 g.

2. 3-Amino-2,5-dichlor-4-methylbenzoesäure

43 g 2,5-Dichlor-4-methyl-3-nitrobenzoesäure und 108,8 g $Na_2S_2O_4$ werden in einem Gemisch aus 340 ml Glykolmonomethylether und 340 ml Wasser 3 Stunden gekocht. In die noch warme Lösung gibt man 480 ml 1/2 konz. HCl und kocht dann noch einmal auf. Nach Abkühlen auf Raumtemperatur gießt man in 1 l Eiswasser und stumpft die stark saure Lösung mit $Na_2CO_3$ ab (pH 5). Der ausgefallene Feststoff wird isoliert und getrocknet. Fp. 218-21°, Ausbeute 32,5 g.

3. 3-Amino-2,5-dichlor-4-methylbenzoesäuremethylester

30 g 3-Amino-2,5-dichlor-4-methylbenzoesäure werden in 140 ml MeOH vorgelegt. Man leitet nun 20 Minuten HCl-Gas ein und kocht anschließend noch 5 Stunden am Rückfluß. Nach Abkühlen wird in Wasser gegeben und mit Soda alkalisch gestellt. Der ausgefallene Ester wird isoliert und getrocknet. Ausbeute 30,8 g Fp. 53-55°.

4. 2,5-Dichlor-3-fluor-4-methylbenzoesäure

30 g 3-Amino-2,5-dichlor-4-methylbenzoesäure-methylester werden in wäßriger Lösung mit $NaNO_2$/HCl ins Diazoniumsalz überführt. Zu der Diazoniumsalzlösung gibt man bei 0°C 33 ml 30 %ige $HBF_4$. Nach 30 Minuten bei 0°C wird der gebildete Feststoff isoliert, mit 5 %iger $HBF_4$-Lösung gewaschen und über $P_2O_5$ getrocknet.

Das Diazoniumsalz wird im Glaskolben trocken erhitzt. Nach beendeter Reaktion wird rohe 2,5-Dichlor-3-fluor-4-methylbenzoesäuremethylester in 100 ml 50 %igen EtOH aufgenommen und mit 8,2 g KOH versetzt. Man kocht 1 Stunde am Rückfluß und säuert nach Abkühlen auf Raumtemperatur mit HCl an. Die ausgefallene Benzoesäure wird isoliert, getrocknet und aus Toluol umkristallisiert. Ausbeute 11,3 g, Fp. 195-6°.

Darstellung von 3,5-Difluor-4-methyl-benzoylchlorid

520 g 3-Fluor-4-methyl-5-nitro-benzoesäure werden in 3500 ml Dioxan mit 50 g Katalysator Pd-C (5 %ig) bei 50°C und 30 bar hydriert. Anschließend wird vom Katalysator abgesaugt und die Lösung eingeengt. Man erhält 403 g 3-Fluor-4-methyl-5-amino-benzoesäure als Rohware.

Die Rohware wird in 950 ml Flußsäure, wasserfrei bei 0-20°C eingetragen. Anschließend werden bei 0-5°C portionsweise 196 g Natriumnitrit zugegeben. Man rührt 30 Minuten bei 0°C nach, läßt 500 ml Dimethylsulfoxid zulaufen und heizt langsam nach, wobei bei etwa 30°C die Stickstoffabspaltung beginnt. Bei 80-85°C wird solange nachgerührt, bis die $N_2$-Abspaltung beendet ist. Der Ansatz wird abgekühlt, auf Eis gegeben und die Kristalle abgesaugt. Nach Lösen in verdünnter NaOH wird vom unlöslichen Anteil abfiltriert und mit Salzsäure wieder sauer gestellt. Die ausgefallenen Kristalle werden abgesaugt, gewaschen und getrocknet. Man erhält 293 g 3,5-Difluor-4-methyl-benzoesäure als Rohware. Nach Zugabe von 380 ml Thionylchlorid wird langsam bis auf Rückflußtemperatur geheizt und solange gerührt, bis die Gasentwicklung beendet ist. Das überschüssige Thionylchlorid wird abdestilliert, der Rückstand grob überdestilliert, das Destillat fraktioniert. Man erhält 137 g 3,5-Difluor-4-methyl-benzoylchlorid vom Kp: 87-88°C/18 mbar $n_D^{20}$: 1,5132.

Durch Hydrolyse einer Probe erhält man die freie 3,5-Difluor-4-methyl-benzoesäure als Kristalle vom Schmelzpunkt Fp: 153°C.

Darstellung von 3,5-Difluor-4-methyl-6-chlor-benzoylchlorid

In 70 g 3,5-Difluor-4-methyl-benzoylchlorid und einer Spatelspitze FeS und $I_2$ werden bei 40-45° unter leichter Kühlung 30 g Chlor eingeleitet. Anschließend wird mit Stickstoff ausgeblasen, der Rückstand grob destilliert und das Destillat fraktioniert. Man erhält 44 g 3,5-Difluor-4-methyl-6-chlorbenzoylchlorid vom Kp: 109°/16 mbar $n_D^{20}$: 1,5342. Aus dem Nachlauf wird das 3,5-Difluor-2,6-dichlor-4-methyl-benzoylchlorid vom Siedepunkt Kp: 109°/13 mbar, $n_D^{20}$: 1,5274 erhalten.

Darstellung von 2-Nitro-4-methyl-5-fluor-benzoesäure

50 g 3-Fluor-4-methyl-benzoesäure werden in 100 ml Schwefelsäure konz. vorgelegt und bei 20-25° unter Eiskühlung eine Mischung bestehend aus 26 g Salpetersäure 98 %ig, und 40 g Schwefelsäure, konz. zugetropft. Man läßt 1 Stunde bei Raumtemperatur nachrühren. Anschließend wird der Ansatz auf Eiswasser gegeben, die Kristalle abgesaugt und gründlich mit Wasser gewaschen. Nach dem Trocknen und Umkristallisation aus Toluol werden 48 g (74,3 % d. Th.) 2-Nitro-4-methyl-5-fluorbenzoesäure vom Schmelzpunkt Fp: 163-5°C erhalten.

Darstellung von 2-Amino-4-methyl-5-fluor-benzoesäure

525 g 2-Nitro-4-methyl-5-fluor-benzoesäure werden in 3 l Dioxan mit 50 g Katalysator Pd-C 5 %ig bei 30-40° und 20-30 bar hydriert. Es wird vom Katalysator abgesaugt und die Lösung auf Wasser gegeben. Die ausgefallenen Kristalle werden abgesaugt und getrocknet.
Ausbeute 357 g (80,1 % d. Th.), Fp.: 201-202°C.

Darstellung von 2,5-Difluor-4-methyl-benzoesäure

Es werden 850 ml Flußsäure, wasserfrei vorgelegt und bei 0-20°C 352 g 2-Amino-4-methyl-5-fluor-benzoesäure eingetragen. Anschließend werden bei 0-5°C portionsweise 173 g Natriumnitrit in ca. 60 Minuten eingetragen. Man läßt 1 Stunde bei 0°C nachrühren, dann wird langsam auf 40° geheizt. Es werden 850 ml Dimethylsulfoxid zugetropft und weiter aufgeheizt. Bei 90-100° wird bis zum Ende der Stickstoffabspaltung gerührt, dann abgekühlt und auf Eis gegeben. Die Kristalle werden abgesaugt und getrocknet.
Ausbeute: 250 g, Fp: 152-8 °C.
Aus Toluol: 162 g, 2,5-Difluor-4-methyl-benzoesäure vom Schmelzpunkt Fp. 160°C.

Darstellung von 2,5-Difluor-4-methyl-benzoylchlorid

250 ml Thionylchlorid werden vorgelegt und bei Raumtemperatur 120 g 2,5-Difluor-4-methyl-benzoesäure

portionsweise eingetragen, zügige Gasentwicklung. Nach Abklingen der Gasentwicklung wird langsam bis auf Rückflußtemperatur geheizt und solange gerührt, bis die Gasentwicklung beendet ist. Das überschüssige Thionylchlorid wird abdestilliert, das Reaktionsprodukt grob überdestilliert und an kleiner Kolonne fraktioniert. Ausbeute 97 g (72,9 % d. Th.) 2,5-Difluor-4-methylbenzoylchlorid vom Siedepunkt Kp: 103°/20 mbar $n_D^{20}$: 1,5232.

## Patentansprüche

1. Alkyl-1-cyclopropyl-1,4-dihydro-4-oxo-3-chinolincarbonsäuren der Formel I

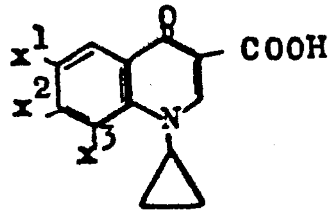

(I)

in welcher

$X^1$,$X^2$ und $X^3$ Wasserstoff und/oder einen Alkylrest mit 1 bis 3 Kohlenstoffatomen bedeuten, mit der Maßgabe, daß ein Rest $X^1$,$X^2$ oder $X^3$ einen Alkylrest darstellt, und ferner $X^1$, $X^2$ und/oder $X^3$ die Nitrogruppe oder ein Halogenatom sein können

und deren pharmazeutisch verwendbare Salze.

2. Alkyl-1-cyclopropyl-1,4-dihydro-4-oxo-3-chinolincarbonsäuren aus der Gruppe bestehend aus den Verbindungen 1-Cyclopropyl-6,8-difluor-7-methyl-1,4-dihydro-4-oxo-3-chinolincarbonsäure, 1-Cyclopropyl-6-fluor-7-methyl-1,4-dihydro-4-oxo-3-chinolincarbonsäure, 1-Cyclopropyl-6-chlor-7-methyl-1,4-dihydro-4-oxo-3-chinolincarbonsäure,

7-Chlor-1-cyclopropyl-8-methyl-1,4-dihydro-4-oxo-3-chinolincarbonsäure, 8-Chlor-1-cyclopropyl-6-fluor-7-methyl-1,4-dihydro-4-oxo-3-chinolincarbonsäure, 6-Chlor-1-cyclopropyl-8-fluor-7-methyl-1,4-dihydro-4-oxo-3-chinolincarbonsäure, 7-Chlor-1-cyclopropyl-6-fluor-8-methyl-1,4-dihydro-4-oxo-3-chinolincarbonsäure, 1-Cyclopropyl-6-fluor-7-methyl-8-nitro-1,4-dihydro-4-oxo-3-chinolincarbonsäure, 1-Cyclopropyl-7-methyl-6,8-dinitro-1,4-dihydro-4-oxo-3-chinolincarbonsäure, 1-Cyclopropyl-6-methyl-7-nitro-1,4-dihydro-4-oxo-3-chinolincarbonsäure, 6-Chlor-1-cyclopropyl-7-methyl-8-nitro-1,4-dihydro-4-oxo-3-chinolincarbonsäure, 7-Chlor-1-cyclopropyl-6-methyl-1,4-dihydro-4-oxo-3-chinolincarbonsäure, 1-Cyclopropyl-6,7-dimethyl-1,4-dihydro-4-oxo-3-chinolincarbonsäure, 1-Cyclopropyl-6,7-dimethyl-8-nitro-1,4-dihydro-4-oxo-3-chinolincarbonsäure, 1-Cyclopropyl-7-methyl-6-nitro-1,4-dihydro-4-oxo-3-chinolincarbonsäure und deren pharmazeutisch verwendbaren Alkalisalzen, Erdalkalisalzen oder Hydraten.

3. Verfahren zur Herstellung von Alkyl-1-cyclopropyl-1,4-dihydro-4-oxo-3-chinolincarbonsäuren der Formel (I)

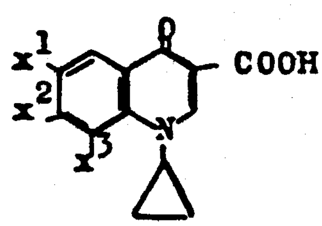

(I)

worin

$X^1$, $X^2$ und $X^3$ Wasserstoff und/oder einen Alkylrest mit 1 bis 3 Kohlenstoffatomen bedeuten mit der Maßgabe, daß ein Rest $X^1$, $X^2$ oder $X^3$ einen Alkylrest darstellt, und ferner $X^1$, $X^2$ und/oder $X^3$ die Nitrogruppe oder ein Halogenatom sein können

und deren pharmazeutisch verwendbaren Salze, dadurch gekennzeichnet, daß man substituierte Benzoesäuren der Formel (1)

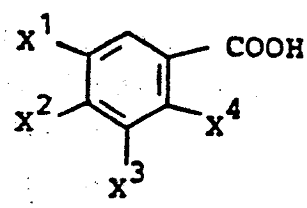

(1)

in der

$X^4$ für Halogen und Nitro steht und

$X^1$, $X^2$, $X^3$ die oben angegebenen Bedeutungen habne,

in das entsprechende Säurehalogenid der Formel (2)

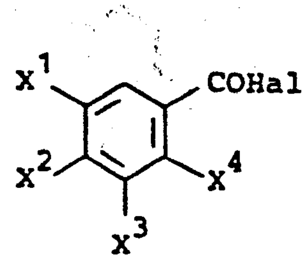

(2)

0181588

worin

$X^1, X^2, X^3$ und $X^4$ wie oben definiert sind und

Hal für Halogen steht, überführt,

Malonsäurediethylester in Gegenwart von Magnesiummethylat mit dem substituierten Benzoylhalogenid (2) zum Aroylmalonester der Formel (3)

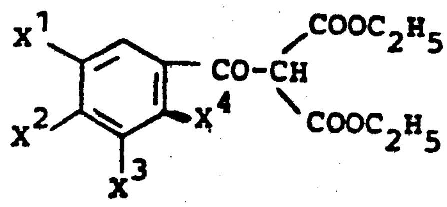

(3)

acyliert, wobei

$X^1, X^2, X^3$ und $X^4$ wieder wie oben definiert sind,

den Aroylmalonester (3) durch partielle Verseifung und Decarboxylierung in wäßrigem Medium mit katalytischen Mengen Schwefelsäure oder p-Toluolsulfonsäure in den Aroylessigsäureethylester (4)

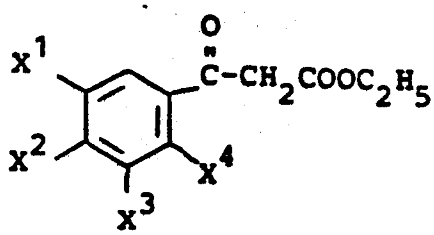

(4)

überführt, wobei

$X^1$, $X^2$, $X^3$ und $X^4$ wieder wie oben definiert sind,

den Aroylessigsäureethylester mit o-Ameisentriethylester/Acetanhydrid zum substituierten 3-Ethoxyacrylsäureethylester (5)

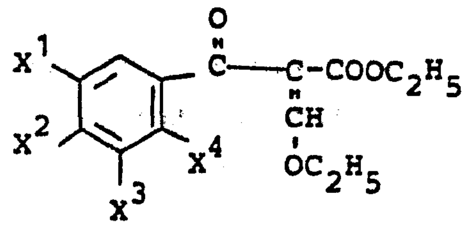

(5)

mit den Definitionen wie oben umsetzt,

das Umsetzungsprodukt (5) mit Cyclopropylamin in einem Lösungsmittel in das Zwischenprodukt (6)

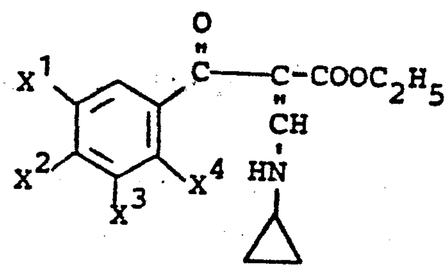

(6)

mit den Substituentendefinitionen wie oben überführt,

das Zwischenprodukt (6) bei Temperaturen von 60 bis 300°C zur Verbindung (7)

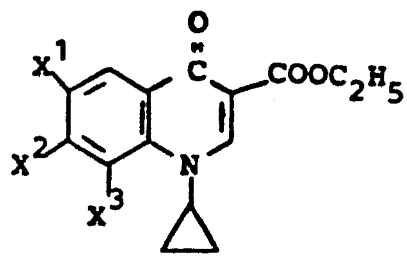

(7)

cyclisiert und schließlich die Verbindung (7) zu Verbindungen der Formel (I) verseift und gegebenenfalls in ein pharmazeutisch verwendbares Salz überführt.

4. Verfahren nach Anspruch 3, dadurch gekennzeichnet, daß $X^4$ für Chlor, Fluor oder Nitro steht.

5. Verfahren nach Anspruch 3 oder 4, dadurch gekennzeichnet, daß die Umsetzung der Verbindung (5) zur Verbindung (6) in Methylenchlorid, Ethylalkohol, Chloroform, Cyclohexan oder Toluol durchgeführt wird.

6. Verfahren nach den Ansprüchen 3, 4 oder 5, dadurch gekennzeichnet, daß man die Umsetzung der Verbindung (6) zur Verbindung (7) in Dimethylsulfoxid, N-Methylpyrrolidon, Sulfolan, Hexamethylphosphorsäuretriamid oder Dimethylformamid durchführt und als Säurebinder Kalium-tert.-butanolat, Butyllithium, Lithium-phenyl, Phenylmagnesiumbromid, Natriummethylat, Natriumhydrid oder Kalium- oder Natriumcarbonat gegebenenfalls in einem Überschuß von 10 Mol-%, verwendet.

7. Arzneimittel, gekennzeichnet durch einen Gehalt an mindestens einer Verbindung der Formel (I)

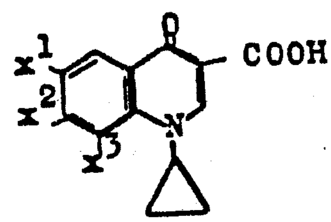

(I)

worin

$X^1$, $X^2$ und $X^3$ Wasserstoff und/oder einen Alkylrest mit 1 bis 3 Kohlenstoffatomen bedeuten, mit der Maßgabe, daß ein Rest $X^1$, $X^2$ oder $X^3$ einen Alkylrest darstellt, und ferner $X^1$, $X^2$ und/oder $X^3$ die Nitrogruppe oder ein Halogenatom sein können

und deren pharmazeutisch verwendbare Salze.

8. Verwendung von Verbindungen der Formel (I)

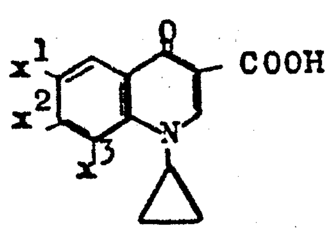

(I)

worin

$X^1$,$X^2$ und $X^3$ Wasserstoff und/oder einen Alkylrest mit 1 bis 3 Kohlenstoffatomen bedeuten, mit der Maßgabe, daß ein Rest $X^1$,$X^2$ oder $X^3$ einen Alkylrest darstellt, und ferner $X^1$, $X^2$ und/oder $X^3$ die Nitrogruppe oder ein Halogenatom sein können

und deren pharmazeutisch verwendbare Salze,

bei der Bekämpfung von bakteriellen Krankheiten oder als Wachstumsförderer im Bereich der Tierernährung.

9. Verwendung von Verbindungen der Formel (I)

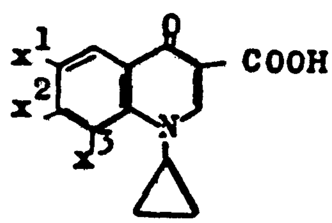

(I)

worin

$X^1$,$X^2$ und $X^3$ Wasserstoff und/oder einen Alkylrest mit 1 bis 3 Kohlenstoffatomen bedeuten, mit der Maßgabe, daß ein Rest $X^1$,$X^2$ oder $X^3$ einen Alkylrest darstellt, und ferner $X^1$, $X^2$ und/oder $X^3$ die Nitrogruppe oder ein Halogenatom sein können

und deren pharmazeutisch verwendbare Salze,

bei der Herstellung von Arzneimitteln oder Wachstumsförderern im Bereich der Tierernährung.

10. Verwendung von Verbindungen aus der Gruppe bestehend aus 1-Cyclopropyl-6,8-difluor-7-methyl-1,4-dihydro-4-oxo-3-chinolincarbonsäure, 1-Cyclopropyl-6-fluor-7-methyl-1,4-dihydro-4-oxo-3-chinolincarbonsäure, 1-Cyclopropyl-6-chlor-7-methyl-1,4-dihydro-4-oxo-3-chinolincarbonsäure, 7-Chlor-1-cyclopropyl-8-methyl-1,4-dihydro-4-oxo-3-chinolincarbonsäure, 8-Chlor-1-cyclopropyl-6-fluor-7-methyl-1,4-dihydro-4-oxo-3-chinolincarbonsäure, 6-Chlor-1-cyclopropyl-8-fluor-7-methyl-1,4-dihydro-4-oxo-3-chinolincarbonsäure, 7-Chlor-1-cyclopropyl-6-fluor-8-methyl-1,4-dihydro-4-oxo-3-chinolincarbonsäure, 1-Cyclopropyl-6-fluor-7-methyl-8-nitro-1,4-dihydro-4-oxo-3-chinolincarbonsäure, 1-Cyclopropyl-7-methyl-6,8-dinitro-1,4-dihydro-4-oxo-3-chinolincarbonsäure, 1-Cyclopropyl-6-methyl-7-nitro-1,4-dihydro-4-oxo-3-chinolincarbonsäure, 6-Chlor-1-cyclopropyl-7-methyl-8-nitro-1,4-dihydro-4-oxo-3-chinolincarbonsäure, 7-Chlor-1-cyclopropyl-6-methyl-1,4-dihydro-4-oxo-3-chinolincarbonsäure, 1-Cyclopropyl-6,7-dimethyl-1,4-dihydro-4-oxo-3-chinolincarbonsäure, 1-Cyclopropyl-6,7-dimethyl-8-nitro-1,4-dihydro-4-oxo-3-chinolincarbonsäure, 1-Cyclopropyl-7-methyl-6-nitro-1,4-dihydro-4-oxo-3-chinolincarbonsäure und deren pharmazeutisch verwendbaren Alkalisalzen, Erdalkalisalzen oder Hydraten bei der Herstellung von Arzneimitteln oder Wachstumsförderern im Bereich der Tierernährung.